# EUROPEAN PATENT SPECIFICATION

(11) **EP 1 096 020 B1**
(45) Date of publication and mention of the grant of the patent: **01.06.2005**
(21) Application number: 00309382.0
(22) Date of filing: 25.10.2000
(51) Int. Cl.: C12P 7/48, C12N 1/14, C12R 1/685

(54) **Process for the manufacture of citric acid**
Verfahren zur Herstellung von Citronensäure
Procédé pour préparation d'acide citrique

(30) Priority: 26.10.1999 GB 9925215
(43) Date of publication of application: 02.05.2001
(73) Proprietor: CERESTAR HOLDING B.V., 4551 LA Sas Van Gent (NL)
(72) Inventor: Troostembergh, Jean-Claude Marie-Pierre Ghislain, 3390 Houwaart (Tielt-Winge) (BE); Oudenne, Francois Catherine Marie Renée, 1082 Brussel (BE); Obyn, Willy Richard, 1910 Kampenhout (BE)
(74) Representative: Knowles, James Atherton

(56) References cited:
- US-A- 4 791 058
- US-A- 5 532 148
- SRINIVAS, M.R.S. ET AL.: "Use of Plackett-Burman design for rapid screening of several nitrogen sources, growth/product promoters, minerals and enzyme inducers for the production of alpha-galactosidase by Aspergillus niger MRSS 234 in solid state fermentation system." BIOPROCESS ENGINEERING, vol. 10, 1994, pages 139-44, XP000906746
- FADEL, M. ET AL.: "Production of cellulases and beta-glucosidase by new isolate of Aspergilus niger F-92." EGYPTIAN JOURNAL OF MICROBIOLOGY, vol. 29, no. 2, 1994, pages 175-82, XP000906700
- INST IND MICROBIOL CITRIC ACID FERMENT GROUP TIANJIN: "STUDIES ON STRAIN SELECTION AND FERMENTATION CONDITIONS OF CITRIC-ACID FROM SWEET-POTATO POWDER" ACTA MICROBIOLOGICA SINICA, vol. 16, no. 2, 1976, pages 166-170, XP002162431 ISSN: 0001-6209
- LU M Y ET AL: "Citric acid production by Aspergillus niger in solid-substrate fermentation." BIORESOURCE TECHNOLOGY, vol. 54, no. 3, 1995, pages 235-239, XP000990029 ISSN: 0960-8524

## Description

### Technical field

The present invention relates to a process for producing and recovering citric acid in a mineral anion-free culture medium. A mineral anion-free nitrogen source such as for example cottonseed meal, soy meal, and/or potato protein is used for the fermentation. The mineral anion-free culture allows a simplified downstream processing. The citric acid is directly recoverable in crystalline form.

### Background of the invention

Carbohydrate substrates are fermented to raw aqueous citric acid solutions using a variety of microorganisms, but in particular yeasts and micro-organisms belonging to the genus *Aspergillus* are applied for producing citric acid. Conventionally the fermentation is followed by a filtration step to remove the mycelium, and a preliminary separation of citric acid from macromolecular contaminants like proteins and non-fermented carbohydrates. Classically the separation of citric acid from the fermentation broth is performed by the addition of calcium hydroxide for precipitating calcium citrate. The overall process requires the use of large amounts of calcium hydroxide and sulphuric acid and results in a disposal problem of large quantities of impure gypsum. Improved downstream processing has been thoroughly investigated.

EP 0 613 878 describes a gypsum-free downstream processing of micro-organism-free citric acid containing fermentation broth. A solvent extraction with a water-immiscible organic solvent, such as trialkyl amine is performed.

DE 195 45 303 describes a gypsum-free and solvent-free process wherein the removal of proteineous material is performed by precipitation with silicium oxide of a particular surface area of at least 500 m²/g. Subsequently the clarified citric acid containing solution is treated on a cation exchange resin and anion exchange resin for removing ions and amino acids, followed by crystallisation of citric acid.

WO 97/10350 describes a gypsum-free and solvent-free process distinguished from the aforementioned process in that the proteineous material is predominantly removed by ultrafiltration. Subsequently, further purification with cation exchange resins as well as anion exchange resins is needed to obtain a substantially mineral anion-free citric acid solution, which is concentrated before crystallisation.

The citric acid fermentation group of Tianjin Institute of Industrial Microbiology (in Acta Microbiologica Sinica vol 16(2) (1976) pp 166-170) describes a mutant strain of A. *niger* (γ-144) and use of sweet potato powder as sole nutrient source for citric acid fermentation.

Lu *et al* (Bioresource Technology vol. 54 (1995) pp 235 - 239) describe use of kumara, taro and potato as substrates for citric acid production by solid state fermentation using *A. niger.*

US-A-4 791 058 discloses grape pomace as a substate for citric acid production using *A*. *niger* as fermentation agent.

M. Srinivas et. al. (in Bioprocess Engineering Vol. 10, 1994, pages 139-144) evaluates in a Plackett-Burman design the growth of *Aspergillus niger* MRSS 234 in presence of urea, corn steep liquor, guar flour or soy bean flour for the production of alpha-galactosidase.

M. Fadel et. al. (in Egyptian Journal of Microbiology Vol. 29, 1994, pages 175-182) describes the production of cellulases and β-glucosidase by a new isolate of *Aspergillus niger.* Experiments in presence of soy bean flour as the sole nitrogen source have been evaluated.

EP 0 151 470 describes a process for obtaining citric acid or its salts from solutions which have been obtained by fermentation from carbohydrate-containing raw materials, characterised in that higher molecular weight impurities are separated by membrane filtration, preferably ultrafiltration, and additional adsorption on a non-ionic adsorber resin, e.g. based on polystyrene or polyacrylate, and the citric acid is crystallised after concentration of the solution from which the lower molecular salts have previously been bound with the help of cationic or anionic exchangers or have been removed by precipitation of citric acid as calcium salt.

A further need exists to have in essence a gypsum-free and solvent-free process wherein the fermentation for producing citric acid is requiring less mineral salts resulting in a purer fermentation broth, and consequently after removal of the micro-organism the syrup can directly be concentrated to crystallise citric acid. In general, a need exists for a process wherein the purification steps following fermentation are simplified.

### Summary of the Invention

The present invention discloses a process for producing citric acid by fermentation using micro-organisms and recovering citric acid crystals characterised in that said process comprises the following steps:
a) preparing a mineral anion-free fermentation culture medium containing less than 10 mmoles/L anions,
b) inoculating the fermentation culture with micro-organisms,
c) allowing the micro-organisms to grow until at least 100 g/L citric acid is obtained in the fermentation medium,
d) removing the micro-organisms from the fermentation medium thereby obtaining a micro-organism-free fermentation broth,
e) optionally purifying the micro-organism-free fermentation broth by membrane filtration and/or adsorption on active carbon,
f) concentrating the micro-organism-free fermentation broth, and
g) recovering citric acid crystals by crystallisation from the concentrated micro-organism free fermentation broth.

The present invention further discloses a process which is comprises the following steps:
a) preparing a mineral anion-free fermentation culture medium containing a carbon-source and a nitrogen-source,
b) inoculating the fermentation culture medium with micro-organisms,
c) allowing the micro-organisms to grow until at least 100 g/L citric acid is obtained in the fermentation medium,
d) removing the micro-organisms from the fermentation medium by filtration for obtaining a micro-organism-free fermentation broth,
e) purifying the micro-organism-free fermentation broth by membrane filtration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

The present invention provides a process wherein the micro-organism is belonging to the genus *Aspergillus,* preferably *Aspergillus niger,* more preferably *Aspergillus niger* TCV392.1 deposited under the Budapest Treaty at BCCM/MUCL (Belgian Coordinated Collections of Micro-organisms/ Mycotheque de l'Université Catholique de Louvain) on 02/07/1999 under number MUCL 41921.

The present invention describes a process wherein the mineral anion-free medium contains less than 10 mmoles/L anions, preferably below 5 mmoles/L anions.

The present invention further describes a process wherein the fermentation culture medium for producing citric acid is prepared with a carbon-source and a mineral anion-free nitrogen source selected from the group consisting of cottonseed meal, soy meal, potato protein, and mixtures thereof, optionally containing ammonium sulphate, and/or ammonium nitrate.

Furthermore, the current invention describes a process wherein the micro-organism is allowed to growth in a bubbled column or airlift reactor.

The present invention discloses a process wherein the membrane filtration is ultrafiltration, nanofiltration or reverse osmosis.

The present invention further discloses a process comprising the following steps:
a) preparing a mineral anion-free fermentation culture medium containing as carbon source starch hydrolysate and as mineral anion-free nitrogen source cottonseed meal,
b) inoculating the fermentation culture medium with *Aspergillus* niger TCV392.1 deposited at BCCM/MUCL on 02/07/1999 under number MUCL 41921,
c) allowing *Aspergillus* niger TCV392.1 to grow in a bubbled column or airlift reactor until at least 100 g/L citric acid is obtained in the fermentation medium,
d) removing *Aspergillus* niger TCV392.1 from the fermentation medium by filtration for obtaining a micro-organism-free fermentation broth,
e) purifying the micro-organism-free fermentation broth by ultrafiltration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

The present invention provides a citric acid producing *Aspergillus* strain which is capable of growing in a fermentation culture medium in the presence of a mineral anion-free nitrogen source containing at most 15 mmoles/L anions, and wherein said nitrogen source is selected from the group consisting of cottonseed meal, soy meal, potato protein, and mixtures thereof, optionally containing ammonium sulphate and/or ammonium nitrate.

The present invention further provides a citric acid producing *Aspergillus niger* strain, preferably *Aspergillus niger* TCV392.1 deposited at BCCM/MUCL on 02/07/1999 under number MUCL 41921.

### Detailed description of the invention

The present invention discloses a fermentation process for producing citric acid in a mineral anion-free medium, and results in a simplified downstream processing.

The present invention discloses a process for producing citric acid by fermentation using micro-organisms and recovering citric acid crystals characterised in that said process comprises the following steps:
a) preparing a mineral anion-free fermentation culture medium,
b) inoculating the fermentation culture medium with micro-organisms,
c) allowing the micro-organisms to grow until at least 100 g/L citric acid is obtained in the fermentation medium,
d) removing the micro-organisms from the fermentation medium thereby obtaining a micro-organism-free fermentation broth,
e) optionally purifying the micro-organism-free fermentation broth,
f) concentrating the micro-organism-free fermentation broth, and
g) recovering citric acid crystals by crystallisation from the concentrated micro-organism free fermentation broth.

The present invention further discloses a process which comprises the following steps:
a) preparing a mineral anion-free fermentation culture medium containing a carbon-source and a nitrogen-source,
b) inoculating the fermentation culture medium with micro-organisms,
c) allowing the micro-organisms to grow until at least 100 g/L citric acid is obtained in the fermentation medium,
d) removing the micro-organisms from the fermentation medium by filtration thereby obtaining a micro-organism-free fermentation broth,
e) purifying the micro-organism-free fermentation broth by membrane filtration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

The process of the present invention is performed with fungi, producing citric acid in a mineral anion-free culture medium. It is performed with fungi of the genus *Aspergillus,* and a typically used species is *Aspergillus niger,* preferably a mutant strain of *Aspergillus niger,* i.e *Aspergillus niger* TCV392.1 deposited at BCCM/MUCL on 02/07/1999 under number MUCL 41921.

Basically, two methods can be distinguished for strain selection. In the first method, acid production is tested for individual colonies obtained from single spores (single spore method). Such a method requires automated screening procedures that enable testing of thousands of colonies and is therefore usually done by plate tests. A pH indicator is commonly included in the medium to estimate acid production, and the discrimination of citric acid among other acids is obtained by using p-dimethylaminobenzaldehyde, which specifically measures citric acid. Yields are evaluated by determining the ratio between acid zone and colony diameter. The second method comprises selection of mutants with a specific trait from a large population using a suitable discriminative growth condition (passage method). Selection may be on the basis of resistance against an antimetabolite, or failure to grow on a particular carbon source. Mutants can arise spontaneously or be produced by mutagenic treatment. A variety of methods are used for mutagenesis including exposure to UV light, γ- and X-ray radiation, exposure to chemicals such as nitrous acid, ethyl methane sulphonate (EMS), diethyl sulphate, N-methyl-N'-nitrosoguanidine (NTG) treatment, acridine treatment and the like. A serious drawback of mutagenic treatment is that high doses increase the chances of obtaining more than one mutation per genome. Thus, in addition to a mutation that results in improved citric acid production, an isolate may have other mutations that might, for example, result in slightly reduced viability. To minimise the chances to introduce such unwanted mutations, mutagenic treatment should be performed in such a way that a high percentage of survival is obtained. When a better producing mutant is isolated it should be maintained in a proper way to prevent decay, i.e. lose its particular characteristics favourable for citric acid production. Decay is most pronounced during the vegetative stage and therefore storage of spores is the best way to preserve a strain.

The citric acid producing mutant strains able to growth in mineral anion-free media are obtained by UV irradiation of a strain *Aspergillus niger* TCV 240 (available in our collection). A spore suspension of *Aspergillus niger* TCV 240 is plated on solid medium and irradiated with UV-light to achieve 99% lethality. After incubation the small colonies showing the largest halo were selected and the mutant strain *Aspergillus niger* TCV 392.1 deposited at BCCM/MUCL on 02/07/1999 under number MUCL 41921 is isolated.

The present invention describes a process wherein the mineral anion-free medium contains less than 10 mmoles/L anions, preferably below 5 mmoles/L anions.

The fermentation for producing citric acid is performed with considerable quantities of mineral salts added as nutrients. A number of divalent metal ions have been suggested as being required in limiting amounts for a successful citric acid process. These can include iron, copper, zinc, manganese and magnesium. Ammonium sulphate and/or ammonium nitrate are frequently used as sole nitrogen source for producing citric acid, and the quantity of anions amounts up to 30 to 90 mmoles/L. For obtaining pure citric acid crystals, these salts have to be removed and production costs are increasing accordingly.

The present invention further describes a process wherein the fermentation culture medium for producing citric acid is prepared with a carbon-source and a mineral anion-free nitrogen source containing at most 15 mmoles/L and wherein said nitrogen-source is selected from the group consisting of cottonseed meal, soy meal, potato protein, and mixtures thereof, optionally containing ammonium sulphate, and/or ammonium nitrate, preferably cottonseed meal.

When applying such a mineral anion-free nitrogen source the mineral anion concentration in the fermentation culture medium is kept low. To evaluate whether the culture medium is free from mineral anions the total anion concentration is taken into account, i.e. the soluble anions coming from the nitrogen source as well as from the carbon source. The concentration of the anions is determined by chromatography applying a Dionex system, which is measuring the conductivity of anions in an eluent, based on sodium hydrogen carbonate and sodium carbonate. The used Dionex system is very sensitive for organic material and the column deteriorates by injecting an excess of organic acids. The samples are neutralised and ion-concentrations in the range of 1 to 100 ppm are measured. The anion concentration is determined after sterilisation and before inoculation. The culture medium with cottonseed meal as nitrogen source has 1.74 to 4.55 mmoles/L anions present, the culture medium with soy protein has a content of 3.56 mmoles/L anions, and the culture medium with potato protein has a content of 1.49 to 3.89 mmoles/L anions.

The fermentation culture medium can be prepared with a starch-based carbon source and a mineral anion-free nitrogen source containing at most 10 mmoles/L anions. A nitrogen source including ammonium sulphate and/or ammonium nitrate is allowed as long as the concentration of the anions is not exceeding 10 mmoles/L.

The fermentation broth free from mineral anions results in considerably simplified downstream processing. After the fermentation process is completed the mycelium containing broth can be heated to a temperature of about 60 to 70°C to inactivate the biomass and to obtain partial coagulation of proteins and then centrifuged or filtered, usually by means of the continuous filters, e.g. rotating vacuum drum filters or belt discharge filters. Optionally filter aids may be used to improve filtration.

Subsequent to the removal of the micro-organisms and depending on the final concentration of citric acid and the overall purity of the micro-organism-free fermentation broth, direct concentration can be applied, followed by crystallisation of citric acid, and recovery of citric acid crystals in the form of citric acid monohydrate or anhydrous citric acid. Alternatively the micro-organism-free fermentation broth can be purified by membrane filtration such as ultrafiltration, nanofiltration or reverse osmosis with membranes capable of retaining macromolecular substances, such as proteineous material and polysaccharides, having a molecular weight greater than 10,000 Dalton. The membranes with the cut-off of 10,000 Dalton allow the product to pass through to the raffinate stream (= permeate stream), while the retentate stream contains most of peptides and proteins from the broth.

Subsequently the raffinate can be treated by active carbon for decolorisation. The function of the colour removal step is to yield an aesthetically acceptable i.e. white food grade product. While colour can be removed with resin applications, active carbon is the preferred adsorber. Treatment can be either in a fixed bed system, or by simply adding fine activated carbon directly to the liquor. After a calculated residence time in contact with the activated carbon, the latter is then removed by filtration.

The thus obtained colourless raffinate is further purified by ion exchange treatment. According to the present invention it is especially this treatment with ion exchange resins, which is simplified. Normally ion exchange treatment consists of treatment with cation and anion exchange resins, eventually with double-pass. The current invention allows that the ion exchange treatment consists of the treatment with cation exchange resins, without the addition of the anion exchange step. The purification with anion exchange resins is not necessary because the micro-organisms are allowed to grow in a mineral anion-free culture medium, and especially the concentration of anions (such as sulphate, nitrate, phosphate, chloride, and the like) is extremely low. Avoiding this treatment with anion exchange resins has a significant consequence for the recovery of citric acid. Anion exchange resins, weak anion as well as strong anion exchange resins are known to be good adsorbers of citric acid from citric acid containing media. Either significant citric acid losses are observed during treatment with anion exchange resins or high quantities of desorbent, such as water are required to completely de-adsorb citric acid, resulting in highly diluted solutions. The periodical regeneration of the ion exchange resins by inorganic bases may also be a source of unwanted effluent wastes.

The refined or unrefined micro-organism free citric acid containing syrup is concentrated and citric acid is recovered by crystallisation in the form of citric acid monohydrate or anhydrous crystals. The mother liquor produced from the crystallisers can be recycled through an adsorptive ion exchange unit, or utilised for the production of sodium citrate.

In the present invention the morphology of the applied mycelium is such that the micro-organisms are allowed to grow in a bubbled column or airlift reactor. The airlift reactor refers to configurations where a draft tube is employed to set up a liquid circulation pattern in the vessel. Such a draft tube can be an internal or an external loop. Where the vessel does not have a draft tube, the term bubble column is used.

In submerged culture the morphology of filamentous micro-organisms varies between pellets and free filaments depending on culture and the genotype of the strain. It is clear that the morphological structure is not in itself necessary for a successful yield, but is a result of the correct process parameters. Pellet formation is not necessary, but does give a broth with a lower energy requirement for oxygen transfer. When a change to a filamentous growth type occurs, the dissolved oxygen level may fall by 50 per cent for a fixed input. Diffusion characteristics of pellets versus filamentous mycelium would suggest that while yields may be similar, productivity should be greater without the additional diffusional constraint of pellets.

It has been shown that oxygen acts as a direct regulator of citric acid accumulation as it is favoured by increasing the dissolved oxygen tension of the fermentation medium. Aeration is a significant factor for the productivity of the fermentation process. The industrial practice is to use relatively low aeration rates e.g. 0.1 to 0.5 vvm. Such aeration rates might lead to foaming and e.g. addition of antifoam agents such as silicone can minimise the problem.

Culture pH can have a profound effect on citric acid production, since certain enzymes within the citric acid cycle are pH sensitive. The maintenance of a low pH during fermentation is vital for a good yield and it is generally considered necessary for the pH to fall around pH 2 within a few hours of the initiation of the process, otherwise the yields are reduced. The low pH suppresses both the productions of oxalic acid, which would be toxic, and gluconic acid, which has a significantly higher production rate from sugar than citric acid. In the current invention especially strains are applied which do not produce oxalic acid, and consequently the fermentation can be started at pH values higher than 2, preferably pH value around 4 to 4.5. By starting the fermentation at this higher pH level the addition of sulphuric acid can be omitted, and has indirectly a consequence on the mineral salt content of the fermentation broth. Foreign ions such as sulphate ions coming from sulphuric acid are not present and do not have to be removed in the downstream processing. Normally these sulphate ions increase the content of anions and finally treatment with anion exchange resin would be required.

Furthermore the pH is not allowed to decrease below 2 in order to maintain conditions for maximum activity of the glucoamylase. During the fermentation of the current invention the pH can be controlled by the addition of sodium citrate, thereby avoiding addition of foreign anions, and this contributes to the simplified downstream processing. The fermentation can be started at pH 4.0 to 4.5, and during the course of the fermentation the pH is decreasing until about 2.

With respect to the carbon source, good citric acid yields have been obtained using a starch-based carbon-source such as pure starch (potatoes, wheat or maize) hydrolysed to 10-100 DE with α-amylase. The applied micro-organism has the ability to produce its own amylolytic enzymes which help in the saccharification of starch hydrolysate to the available sugars.

Typically the current invention describes a process for producing citric acid by fermentation and recovering citric acid crystals and said process comprises the following steps:
a) preparing a mineral anion-free fermentation culture medium containing as carbon source starch hydrolysate and as mineral anion-free nitrogen source cottonseed meal,
b) inoculating the fermentation culture medium with *Aspergillus* niger TCV392.1 deposited at BCCM/MUCL on 02/07/1999 under number MUCL 41921,
c) allowing *Aspergillus* niger TCV392.1 to grow in a bubbled column or airlift reactor until at least 100 g/L citric acid is obtained in the fermentation medium,
d) removing *Aspergillus* niger TCV392.1 from the fermentation medium by filtration thereby obtaining a micro-organism-free fermentation broth,
e) purifying the micro-organism-free fermentation broth by ultrafiltration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

The thus obtained crystals can be dried by applying e.g. a moving bed/fluidized bed unit.

The present invention provides a citric acid producing *Aspergillus* strain which is capable of growing in a fermentation culture medium in the presence of a mineral anion-free nitrogen source containing at most 15 mmoles/L anions, and wherein said nitrogen source is selected from the group consisting of cottonseed meal, soy meal, potato protein, and mixtures thereof, optionally containing ammonium sulphate and/or ammonium nitrate.

The present invention further provides a citric acid producing *Aspergillus niger* strain, preferably *Aspergillus niger* TCV392.1 deposited at BCCM/MUCL on 02/07/1999 under number MUCL 41921.

Example 1 describes a mutagenesis method for obtaining *Aspergillus niger* TCV 392.1 deposited at BCCM/MUCL on 02/07/1999 under number MUCL 41921.

Example 2 describes fermentation with cottonseed meal as nitrogen source and the fermentation is followed by measurement of total reducing sugars (TRS), reducing sugars (RS), glucose, polyol content, and citric acid concentration.

The reducing sugars are determined by titration with Fehling reagens, while the total reducing sugars are measured by Fehling reagent after complete hydrolysis of the oligosaccharides. Glucose is determined by measuring in an amperometric electrode system the hydrogen peroxide concentration formed by glucose-oxidase reaction with glucose present in the sample. The citric acid concentration is determined either by titration or by an enzymatic method. The total acidity is determined by titration with sodium hydroxide (0.1 N) in presence of phenolphtalein indicator. The enzymatic method is based on the reaction with citrate lyase, and applying the UV Boehringer kit (No 139076). In presence of cottonseed meal 170 g/L citric acid is produced within 122 hours, while TRS is about 5 g/L, and less than 1 g/L RS.

Example 3 describes the fermentation for producing citric acid with soy meal as nitrogen source, whereby 159 g/L citric acid is formed. In presence of potato protein (example 4) longer fermentation times are needed; 146 hours instead of 122 hours with cottonseed meal.

Example 5 describes the downstream processing of the fermentation process. The purity of citric acid crystals can be determined by measuring readily carbonisable substances (RCS) according to US Pharmacopoeia XXI citric acid method. The crystalline product fulfils US Pharmacopoeia XXI requirements.

The current process has the following advantages:
a) Fermentation can be performed in bubbled column reactor or airlift reactor, which requires low energy input and results in significant lower production costs.
b) Fermentation can be performed in presence of cheap mineral anion-free nitrogen source such as cottonseed meal, soy meal, and/or potato protein, preferably cottonseed meal, thus resulting in a low content of mineral anions of less than 15 mmoles/L, preferably less than 10 mmoles/L, more preferably below 5 mmoles/L.
c) Fermentation can be started at pH 4.5, thus avoiding addition of sulphate ions
d) During fermentation no oxalic acid is formed.
e) pH monitoring can be obtained by the addition of sodium citrate, thus avoiding the addition of foreign anions.
f) Starch hydrolysate is used as carbon source.
g) Optionally purification of micro-organism free fermentation broth and/or raffinate (= permeate) coming from ultrafiltration is using only cation exchange resin optionally preceded by adsorption on activated carbon. The purification is devoid of treatment with anion exchange resins.

The present invention is illustrated by way of the following examples.

### Example 1 - Mutation for obtaining Aspergillus niger TCV 392.1 deposited at BCCM/MUCL on 02/07/1999 under number MUCL 41921

A spore suspension *Aspergillus niger* TCV 240 (available in our collection) was plated on solid medium containing malt extract 6%, calcium carbonate 0.5% and agar 2%, and irradiated with UV-light to achieve 99% lethality. The plates were incubated at 35°C for 5 days. Small colonies showing the largest halo were selected and tested in liquid medium for citric acid production. The strain deposited at BCCM/MUCL on 02/07/1999 under number MUCL 41921 was isolated.

### Example 2 - fermentation in presence of cottonseed meal.

HDx (97 DE High Dextrose Hydrolysate, C 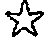 Sweet D02782, d.s = 70%)- syrup was dissolved in de-ionised water until a concentration of 170 g d.s./L was reached. Cottonseed meal (Pharmamedia) was suspended in de-ionised water until 6 g commercial base per liter was dispersed in the fermentation broth. The pH of the suspension was adjusted to 4.5 with citric acid. The fermentation temperature was 37°C and the broth was inoculated with a suspension of *Aspergillus niger* TCV 392.1 (deposited at BCCM/MUCL on 02/07/1999 under number MUCL 41921) spores to reach an initial spores concentration in the range of 50,000 spores/ml. The airflow through the fermenter was automatically adjusted to maintain a dissolved oxygen pressure of 50%. During 1 to 1.5 days of fermentation, foam control with silicone anti-foam was done. The pH decreased from its initial value of 4.5 to about 2.25. In order to maintain the glucoamylase activity of the strain, pH was maintained at this value for the next 2 or 3 days with sodium citrate solution (300 g/L).
122 hours was needed to convert the dextrose into 170 g/L citric acid, measured in the cell-free broth. The fermentation was continued for another 16 to 22 hours to allow further oxidation of part of the residual high sugars without compromising the citric acid content. The cell-free end fermentation broth was containing about 5 g/L TRS, and less than 1 g/L Reducing Sugars (RS), 0.05 g/L glucose.

Table 1 shows the results compared with the other examples.

### Example 3 - fermentation in presence of soy meal

The fermentation conditions were the same as described in example 2, but cottonseed meal was substituted by 7 g commercial base per litre of soymeal (PROVASOY).
After 139 hours fermentation time the final end concentration of citric acid was 159 g/L .

Table 1 shows the results compared with the other examples.

### Example 4 - fermentation in presence of potato protein

The fermentation conditions were as described in example 2, but 7 g (commercial base) per litre potato protein (AKV Kartoffelprotein Koncentraat 33000) was applied instead of cottonseed meal. 146 hours were needed to convert the glucose and 160g/L citric acid was obtained.

Table 1 shows the results compared with the other examples.

**Table 1**

| Nitrogen source | TRS (g/L) | RS (g/L) | Glucose (g/L) | Citric acid (g/L) | Anions Mmoles/L | Fermentation time (h) |
|---|---|---|---|---|---|---|
| Cottonseed meal | 5 | 0.8 | 0.05 | 170 | 4.55 | 122 |
| Soy protein | 4.2 | 2.0 | 0.20 | 159 | 3.56 | 139 |
| Potato protein | 6.4 | 0.9 | 0.0 | 160 | 3.89 | 146 |

### Example 5 -downstream processing of fermentation in presence of cottonseed meal

After fermentation the broth was heated to 60°C for 1 hour in a holding tank to deactivate the biomass.
Then the biomass was removed on a continuous belt filter and in the last section the filter cake was washed with water. An ultrafiltration unit, equipped with polysulphone type membranes of 10,000 Dalton, was applied. The retendate of the ultrafiltration was optionally washed in a diafiltration step, which is part of the ultrafiltration embodiment. The raffinate (=permeate) of the ultrafiltration was fed into a set of columns filled with activated carbon for decolourisation.
Downstream of the carbon columns was a set of strong cation exchanger columns.
The purified liquor was finally concentrated in an evaporator prior to crystallisation.
The crystallisation was performed in two stages, i.e. a crude crystallisation followed by re-crystallisation.

From the first crude crystallisation step massecuite was separated on a centrifuge in a crystal stream and a mother liquor stream. The crystals were sent directly to the dilution tank of the recrystallisation part. The mother liquors of the first crude crystallisation were partly recycled to crystalliser and the remaining part was purged into a trisodium citrate channel.
The crystals from the first crude crystallisation step were re-dissolved in a dilution tank and passed over a set of carbon columns for decolourisation. The liquor from the carbon columns was recrystallised in a third crystalliser.

The wet crystals from the centrifuge at about 3% moisture were dried in a fluid bed drier to remove the residual water to a moisture content of < 0.5%. The overall recovery of citric acid and trisodium citrate was higher than 95%. The purity of citric acid crystals was determined by measuring readily carbonisable substances (RCS) according to US Pharmacopoeia XXI citric acid method. The crystalline product fulfils US Pharmacopoeia XXI requirements.

## Claims

1. A process for producing citric acid by fermentation using micro-organisms and recovering citric acid crystals **characterised in that** said process comprises the following steps:
a) preparing a mineral anion-free fermentation culture medium containing less than 10 mmoles/L anions,
b) inoculating the fermentation culture medium with micro-organisms,
c) allowing the micro-organisms to grow until at least 100 g/L citric acid is obtained in the fermentation medium,
d) removing the micro-organisms from the fermentation medium thereby obtaining a micro-organism-free fermentation broth,
e) optionally purifying the micro-organism-free fermentation broth by membrane filtration and/or adsorption on active carbon,
f) concentrating the micro-organism-free fermentation broth, and
g) recovering citric acid crystals by crystallisation from the concentrated micro-organism free fermentation broth.

2. A process according to claim 1 **characterised in that** said process comprises the following steps:
a) preparing a mineral anion-free fermentation culture medium containing less than 10 mmoles/L anions, the medium containing a carbon-source and a nitrogen-source,
b) inoculating the fermentation culture medium with micro-organisms,
c) allowing the micro-organisms to grow until at least 100 g/L citric acid is obtained in the fermentation medium,
d) removing the micro-organisms from the fermentation medium by filtration for obtaining a micro-organism-free fermentation broth,
e) purifying the micro-organism-free fermentation broth by membrane filtration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

3. A process according to claim 1 or 2 **characterised in that** the micro-organism belongs to the genus *Aspergillus.*

4. A process according to claim 3 **characterised in that** the micro-organism is an *Aspergillus* strain, preferably an *Aspergillus niger,* more preferably the *Aspergillus niger* TCV 392.1 strain deposited at BCCM/MUCL on 02/07/1999 under number MUCL 41921.

5. A process according to any one of claims 1 to 4 **characterised in that** the mineral anion-free medium contains below 5 mmoles/L anions.

6. A process according to any one of claims 1 to 5 **characterised in that** the fermentation culture medium is prepared with a carbon-source and a mineral anion-free nitrogen-source containing at most 10 mmoles/L anions, and wherein said nitrogen source is selected from the group consisting of cottonseed meal, soy meal, potato protein, and mixtures thereof, optionally containing ammonium sulphate, and/or ammonium nitrate.

7. A process according to any one of claims 1 to 6 **characterised in that** the micro-organism is allowed to growth in a bubbled column or airlift reactor.

8. A process according to anyone of claims 2 to 7 **characterised in that** the membrane filtration is ultrafiltration, nanofiltration or reverse osmosis.

9. A process according to any one of claims 1 to 8 **characterised in that** said process comprises the following steps:
a) preparing a mineral anion-free fermentation culture medium containing less than 10 mmoles/L anions, the medium containing as carbon-source starch hydrolysate and as mineral anion-free nitrogen-source cottonseed meal, anion-free nitrogen source cottonseed meal,
b) inoculating the fermentation culture medium with *Aspergillus niger* TCV392.1 deposited at BCCM/MUCL on 02/07/1999 under number MUCL 41921,
c) allowing *Aspergillus niger* TCV392.1 to grow in a bubbled column or airlift reactor until at least 100 g/L citric acid is obtained in the fermentation medium,
d) removing *Aspergillus niger* TCV392.1 from the fermentation medium by filtration thereby obtaining a micro-organism-free fermentation broth,
e) purifying the micro-organism-free fermentation broth by ultrafiltration, and/or adsorption on active carbon,
f) further purifying the micro-organism-free fermentation broth by ion exchange treatment consisting of treatment with cation exchange resin for obtaining a purified fermentation broth,
g) concentrating the purified fermentation broth, and
h) recovering citric acid crystals by crystallisation from the concentrated purified fermentation broth.

10. An *Aspergillus niger* strain TCV392.1 deposited at BCCM/MUCL on 02/07/1999 under number MUCL 41921.

## Patentansprüche

1. Verfahren zur Herstellung von Zitronensäure durch Fermentation unter Verwendung von Mikroorganismen und Gewinnung von Zitronensäurekristallen, **dadurch gekennzeichnet, daß** das Verfahren die folgenden Schritte umfaßt:
a) Herstellung eines Anionen-freien Fermentationskulturmineralmediums, das weniger als 10 mmol/l Anionen enthält;
b) Inokulation des Fermentationskulturmediums mit Mikroorganismen,
c) Ermöglichen, daß die Mikroorganismen wachsen können, bis zumindest 100 g/l Zitronensäure in dem Fermentationsmedium erhalten werden,
d) Entfernung der Mikroorganismen aus dem Fermentationsmedium, wobei eine Mikroorganismen-freie Fermentationsbrühe erhalten wird,
e) optionale Reinigung der Mikroorganismen-freien Fermeatationsbrühe durch Membranfiltration und/oder Adsorption auf Aktivkohle;
f) Konzentration der Mikroorganismen-freien Fermentationsbrühe, und
g) Gewinnung von Zitronensäurekristallen durch Kristallisation aus der konzentrierten Mikroorganismen-freien Fermentationsbrühe.

2. Verfahren nach Anspruch 1, **dadurch gekennzeichnet, daß** das Verfahren die folgenden Schritte umfaßt:
a) Herstellung eines Anionen-freien Fermentationskulturmineralmediums, das weniger als 10 mmol/l Anionen enthält, wobei das Medium eine Kohlenstoffquelle und eine Stickstoffquelle enthält;
b) Inokulation des Fermentationskulturmediums mit Mikroorganismen,
c) Ermöglichen, daß die Mikroorganismen wachsen können, bis zumindest 100 g/l Zitronensäure in dem Fermentationsmedium erhalten werden,
d) Entfernung der Mikroorganismen aus dem Fermentationsmedium, wobei eine Mikroorganismen-freie Fermentationsbrühe erhalten wird,
e) Reinigung der Mikroorganismen-freien Fermentationsbrühe durch Membranfiltration und/oder Adsorption auf Aktivkohle,
f) weitere Reinigung der Mikroorganismen-freien Fermentationsbrühe durch Ionenaustauschbehandlung, bestehend aus der Behandlung mit Kationenaustauschharz für den Erhalt einer gereinigten Fermentationsbrühe;
g) Konzentration der Mikroorganismen-freien Fermentationsbrühe, und
g) Gewinnung von Zitronensäurekristallen durch Kristallisation aus der konzentrierten gereinigten Fermentationsbrühe.

3. Verfahren nach Anspruch 1 oder 2, **dadurch gekennzeichnet, daß** der Mikroorganismus zu der Gattung Aspergillus gehört.

4. Verfahren nach Anspruch 3, **dadurch gekennzeichnet, daß** der Mikroorganismus ein Aspergillus-Stamm ist, bevorzugt ein Aspergillus niger, stärker bevorzugt der Aspergillus niger TCV 392.1-Stamm, der am 02.07.1999 unter der Nummer MUCL 41921 bei der BCCM/MUCL hinterlegt wurde.

5. Verfahren nach einem der Ansprüche 1 bis 4, **dadurch gekennzeichnet, daß** das Anionen-freie Mineralmedium unter 5 mmol/l Anionen enthält.

6. Verfahren nach einem der Ansprüche 1 bis 5, **dadurch gekennzeichnet, daß** das Fermentationskulturmedium mit einer Kohlenstoffquelle und einer mineralischen Anionen-freien Stickstoffquelle hergestellt wird, die höchstens 10 mmol/l Anionen enthält, und worin die Stickstoffquelle aus der Gruppe, bestehend aus Baumwollsaatenmehl, Sojamehl, Kartoffelprotein und Gemischen hiervon, gegebenenfalls enthaltend Ammoniumsulfat und/oder Ammoniumnitrat, ausgewählt ist.

7. Verfahren nach einem der Ansprüche 1 bis 6, **dadurch gekennzeichnet, daß** der Mikroorganismus in einer durchblasenen Säule oder einem Airlift-Reaktor wachsen kann.

8. Verfahren nach einem der Ansprüche 2 bis 7, **dadurch gekennzeichnet, daß** die Membranfiltration Ultrafiltration, Nanofiltration oder Umkehrosmose ist.

9. Verfahren nach einem der Ansprüche 1 bis 8, **dadurch gekennzeichnet, daß** das Verfahren die folgenden Schritte umfaßt:
a) Herstellung eines Anionen-freien Fermentationskulturmineralmediums, enthaltend weniger als 10 mmol/l Anionen, wobei das Medium als Kohlenstoffquelle Stärkehydrolysat und als mineralische Anionen-freie Stickstoffquelle Baumwollsamenmehl enthält,
b) Inokulation des Fermentationskulturmediums mit Aspergillus niger TCV392.1, hinterlegt am 02.07.1999 bei BCCM/MUCL unter der Nummer MUCL 41921;
c) Ermöglichen, daß Aspergillus niger TCV392.1 in einer durchblasenen Säule oder in einem Airlift-Reaktor wachsen kann, bis zumindest 100 g/l Zitronensäure in dem Fermentationsmedium erhalten werden,
d) Entfernung von Aspergillus niger TCV392.1 aus dem Fermentationsmedium durch Filtration, wodurch eine Mikroorganismen-freie Fermentationsbrühe erhalten wird,
e) Reinigung der Mikroorganismen-freien Fermentationsbrühe durch Ultrafiltration und/oder Adsorption auf Aktivkohle,
f) weitere Reinigung der Mikroorganismen-freien Fermentationsbrühe durch Ionenaustaüschbehandlung, bestehend aus der Behandlung mit Kationenaustauschharz, zum Erhalt einer gereinigten Fermentationsbrühe,
g) Konzentration der gereinigten Fermentationsbrühe, und
h) Gewinnung von Zitronensäurekristallen durch Kristallisation aus der konzentrierten gereinigten Fermentationsbrühe.

10. Aspergillus niger-Stamm TCV 392.1, hinterlegt am 02.07.1999 bei BCCM/MUCL unter der Nummer MUCL 41921.

## Revendications

1. Procédé pour la production d'acide citrique par fermentation en utilisant des micro-organismes et en récupérant des cristaux d'acide citrique, **caractérisé en ce que** ledit procédé comprend les étapes suivantes:
a) préparer un milieu de culture de fermentation sans anion minéral contenant moins de 10 mmoles/l d'anions,
b) inoculer le milieu de culture de fermentation avec des micro-organismes,
c) laisser les micro-organismes croître jusqu'à ce qu'au moins 100 g/l d'acide citrique soient obtenus dans le milieu de fermentation,
d) éliminer les micro-organismes du milieu de fermentation afin d'obtenir un bouillon de fermentation sans micro-organisme,
e) purifier éventuellement le bouillon de fermentation sans micro-organisme par filtration sur membrane et/ou adsorption sur charbon actif,
f) concentrer le bouillon de fermentation sans micro-organisme, et
g) récupérer les cristaux d'acide citrique par cristallisation à partir du bouillon de fermentation sans micro-organisme concentré.

2. Procédé selon la revendication 1, **caractérisé en ce que** ledit procédé comprend les étapes suivantes:
a) préparer un milieu de culture de fermentation sans anion minéral contenant moins de 10 mmoles/l d'anions, le milieu contenant une source de carbone et une source d'azote,
b) inoculer le milieu de culture de fermentation avec des micro-organismes,
c) laisser les micro-organismes croître jusqu'à ce qu'au moins 100 g/l d'acide citrique soient obtenus dans le milieu de fermentation,
d) éliminer les micro-organismes du milieu de fermentation par filtration afin d'obtenir un bouillon de fermentation sans micro-organisme,
e) purifier le bouillon de fermentation sans micro-organisme par filtration sur membrane et/ou adsorption sur charbon actif,
f) purifier de manière supplémentaire le bouillon de fermentation sans micro-organisme par un traitement par échange d'ions constitué d'un traitement avec une résine échangeuse de cations afin d'obtenir un bouillon de fermentation purifié,
g) concentrer le bouillon de fermentation purifié, et
h) récupérer les cristaux d'acide citrique par cristallisation à partir du bouillon de fermentation purifié concentré.

3. Procédé selon la revendication 1 ou 2, **caractérisé en ce que** le micro-organisme appartient au genre *Aspergillus.*

4. Procédé selon la revendication 3, **caractérisé en ce que** le micro-organisme est une souche *Aspergillus,* de préférence un *Aspergillus niger,* plus préférentiellement la souche *Aspergillus niger* TCV 392.1 déposée au BCCM/MUCL le 02/07/1999 sous le numéro MUCL 41921.

5. Procédé selon l'une quelconque des revendications 1 à 4, **caractérisé en ce que** le milieu sans anion minéral contient moins de 5 mmoles/l d'anions.

6. Procédé selon l'une quelconque des revendications 1 à 5, **caractérisé en ce que** le milieu de culture de fermentation est préparé avec une source de carbone et une source d'azote sans anion minéral contenant au plus 10 mmoles/l d'anions, et dans lequel ladite source d'azote est choisie à partir du groupe composé de farine de coton, de farine de soja, de protéine de pomme de terre et de mélanges de celles-ci, contenant éventuellement du sulfate d'ammonium et/ou du nitrate d'ammonium.

7. Procédé selon l'une quelconque des revendications 1 à 6, **caractérisé en ce que** le micro-organisme est laissé croître dans une colonne à barbotage ou dans un bioréacteur à agitation par circulation d'air.

8. Procédé selon l'une quelconque des revendications 2 à 7, **caractérisé en ce que** la filtration sur membrane est une ultrafiltration, une nanofiltration ou une osmose inverse.

9. Procédé selon l'une quelconque des revendications 1 à 8, **caractérisé en ce que** ledit procédé comprend les étapes suivantes:
a) préparer un milieu de culture de fermentation sans anion minéral contenant moins de 10 mmoles/l d'anions, le milieu contenant de l'hydrosylate d'amidon en tant que source de carbone et de la farine de coton en tant que source d'azote sans anion minéral,
b) inoculer le milieu de culture de fermentation avec de *l'Aspergillus niger* TCV 392.1 déposée au BCCM/MUCL le 02/07/1999 sous le numéro MUCL 41921,
c) laisser *l'Aspergillus niger* TCV 392.1 croître, dans une colonne à barbotage ou un bioréacteur à agitation par circulation d'air, jusqu'à ce que au moins 100 g/l d'acide citrique soient obtenus dans le milieu de fermentation,
d) éliminer *l'Aspergillus niger* TCV 392.1 du milieu de fermentation par filtration afin d'obtenir un bouillon de fermentation sans micro-organisme,
e) purifier le bouillon de fermentation sans micro-organisme par ultrafiltration et/ou adsorption sur charbon actif,
f) purifier de manière supplémentaire le bouillon de fermentation sans micro-organisme par un traitement par échange d'ions constitué d'un traitement avec une résine échangeuse de cations afin d'obtenir un bouillon de fermentation purifié,
g) concentrer le bouillon de fermentation purifié, et
h) récupérer les cristaux d'acide citrique par cristallisation à partir du bouillon de fermentation purifié concentré.

10. Souche d'*Aspergillus niger* TCV 392.1 déposée au BCCM/MUCL le 02/07/1999 sous le numéro MUCL 41921.
